# EUROPEAN PATENT APPLICATION

(11) **EP 2 177 533 A1**
(43) Date of publication of application: **21.04.2010**
(21) Application number: 08166259.5
(22) Date of filing: 09.10.2008
(51) Int. Cl.: C07K 14/655, A61K 38/31

(54) **Multiple N-methylated cyclic hexapeptides for treatment of neurogenic inflammation**

(71) Applicant: TU München, 80333 München (DE); Szentagothay Regional University Science Center (of Semmelweis University), 1088 Budapest (HU); Medipolis Regional University Science Center (of University Pecs), 7624 Pécs (HU)
(72) Inventor: Horváth, Aniko, 1088 Budapest (HU); Kéri, György, 1088 Budapest (HU); Mandl, József, 1088 Budapest (HU); Pintér, Erika, Dr., 7624 Pécs (HU); Helyes, Zsuzsanna, Dr., 7624 Pécs (HU); Szolcsányi, János, Dr., 7624 Pécs (HU); Kessler, Horst, Prof. Dr., 85747 Garching (DE)
(74) Representative: HOFFMANN EITLE

(57) **Abstract**

The present invention relates to cyclic hexapeptides containing two or more N-alkylations in their backbone. The described cyclopeptides are active as anti-inflammatory agents and/or analgesics and may be used for the treatment of neuropathic pain and/or neurogenic inflammation.

## Description

### 1. FIELD OF THE INVENTION

The present application pertains to new N-methylated cyclic hexapeptides derived from the peptide hormone Somatostatin. These compounds are highly active and metabolically stable compounds for the treatment of neurogenic inflammation and related disorders.

### 2. BACKGROUND

Neuropathic pain is rather resistant to conventional analgesics. Therefore, different approaches must be taken to treat neuropathic pain. One possibility is the use of agents acting at the level of the sensory nerve terminals themselves to inhibit the release of these pro-inflammatory/pro-nociceptive sensory neuropeptides. Such agents may form the basis for the development of a new group of anti-inflammatory and analgesic drugs.

Neurogenic inflammation refers to vasodilation and plasma protein extravasation (oedema formation) induced by pro-inflammatory sensory neuropeptides such as calcitonin gene-related peptide (CGRP) and substance P (SP) released from the activated sensory nerve terminals in the innervated area. These processes are followed by the activation of inflammatory cells later. The neurogenic component, which plays an important role in the pathological mechanisms of several inflammatory diseases e.g. rheumatoid arthritis, allergic contact dermatitis, psoriasis, asthma and inflammatory bowel diseases, cannot be inhibited by the classical non-steroidal anti-inflammatory drugs. Corticosteroids are only effective in very high doses in which they produce severe side effects. SP and CGRP are also involved in neuropathic pain, which is rather resistant to conventional analgesics. Therefore, agents acting at the level of the sensory nerve terminals themselves to inhibit the release of these pro-inflammatory/pro-nociceptive sensory neuropeptides are desired for the development of a new group of anti-inflammatory and analgesic drugs.

Besides the locally released pro-inflammatory sensory neuropeptides somatostatin is also released from the peripheral terminals of the capsaicin-sensitive peptidergic population of primary sensory neurons in response to activation (Szolcsányi et al. (1998) Br. J. Pharmacol. 123, 936-942; Szolcsányi et al. (1998) Br. J. Pharmacol. 125, 916-922; Helyes et al. (2004) Arthr. Rheum, 50, 1677-1685; Bölcskei et al. (2005) Pain 117, 368-376; Helyes et al. (2007) Am. J. Physiol. Lung Cell. Mol. Physiol. 292, L1173-81). It gets into the systemic circulation and inhibits inflammatory and nociceptive processes in rat and mouse experimental models presumably through somatostatin receptor subtypes 1 and/or 4 (sst₁/sst₄; SRIF1 receptor group), while inhibition of hormone secretion is mediated by the sst₂, sst₃ and sst₅ subtypes (SRIF2 receptor group).

Somatostatin is a tetradecapeptide having the structural formula:

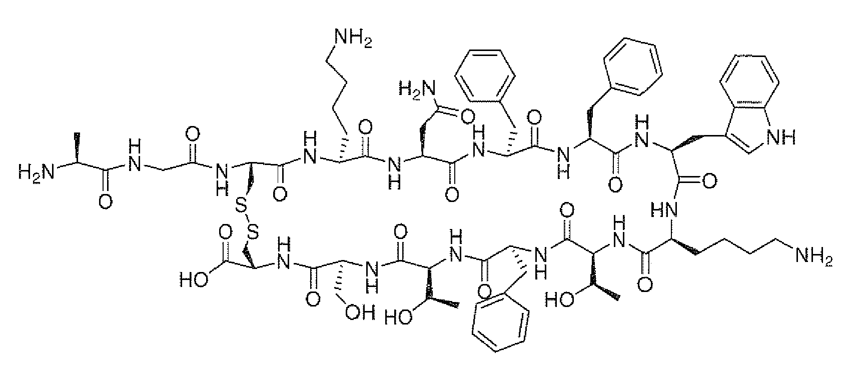

It inhibits growth hormone, insulin, glucagon and also reduces gastric secretions. Although it has interesting biological properties, its therapeutic use has been hampered due to its short half life (<3 min) in serum [G. Weckbecker et al. (2003) Nat. Rev. Drug Discov. 2, 999-1017].

Besides the short plasma elimination half-life, the other problem of native somatostatin concerning its therapeutical application is its wide range of effects, including endocrine actions. These latter actions are considered as undesired side effects if somatostatin is given to inhibit inflammatory or nociceptive processes.

The cyclic hexapeptide, Seglitide (MK-678), which has the structural formula: 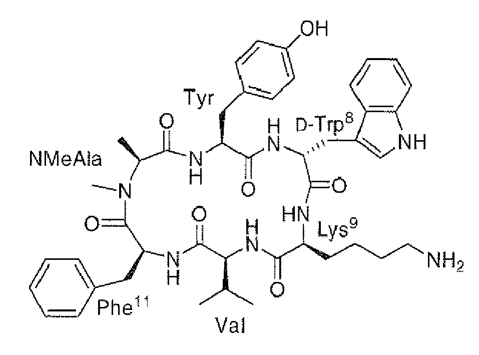 was developed as an analogue of somatostatin. This compound was found to improve control of postprandial hyperglycemia in diabetic animals when given in combination with insulin. The analogue was found to be quite stable in the blood and in the gastrointestinal tract, but the bioavailability after oral administration is only 1-3% [D.F. Veber et al. (1984) Life Sci. 34, 1371-1378].

Having regard to the state of the art discussed above, it is an object of the present invention to provide substances that are effective in the treatment of neuropathic pain and related disorders. More specifically, it is an object of the present invention to provide somatostatin-derived substances that can inhibit inflammation without accompanying endocrine side effects and which exhibit satisfactory half life and bioavailability, especially after oral administration.

### 3. SUMMARY OF THE INVENTION

The present invention pertains to cyclopeptides of formula (I) below: 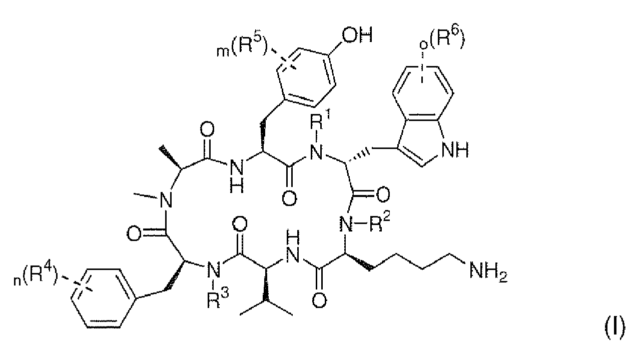
wherein R¹, R², R³ are each independently selected from hydrogen and C₁₋₆ alkyl groups, preferably hydrogen and C₁₋₃ alkyl groups, more preferably hydrogen and methyl groups, with the proviso that at least one of R¹, R², and R³ is not hydrogen; R4, R⁵ and R⁶ are substituents;
n denotes the number of substituents R⁴ present on the benzene ring and is selected from 0, 1, 2, 3, 4, and 5, preferably, 0, 1, 2, 3, and 4, more preferably 0, 1, 2, and 3, further preferably 0, 1, and 2, even more preferably 0 and 1, most preferably 0; if n is 2 or greater, the multiple substituents R⁴ may be the same or different;
m and o denote the respective numbers of substituents R⁵ and R⁶ and are independently selected from 0, 1, 2, 3, and 4, more preferably 0, 1, 2, and 3, further preferably 0, 1, and 2, even more preferably 0 and 1, most preferably 0; if m and/or o are 2 or greater, the multiple R⁵ and/or R⁶ may be the same or different,
and pharmaceutically acceptable salts, hydrates, solvates, polymorphs and pseudopolymorphs thereof.

The present invention furthermore pertains to pharmaceutical compositions and medical uses of the above-mentioned inventive compounds. It pertains in particular to the use of the above compounds as anti-inflammatory agents and/or analgesics in the treatment of neuropathic pain, neurogenic inflammation and related disorders.

The present invention furthermore pertains to methods for manufacturing the compounds of the invention.

### 4. BRIEF EXPLANATION OF THE DRAWINGS

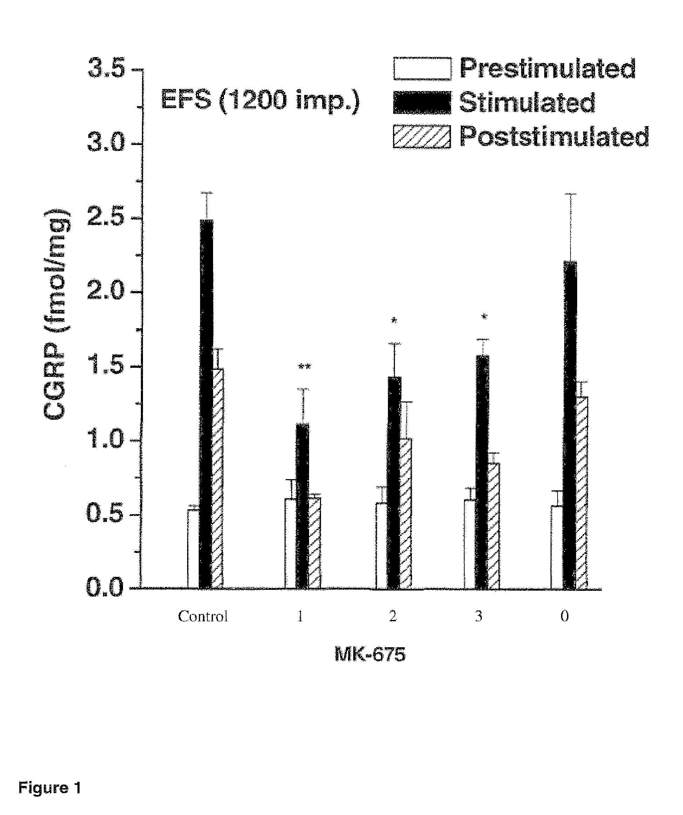

Figure 1 provides a graphical representation of the effect of some compounds of the present invention on electrical field stimulation (EFS; 40 V, 0.1 ms, 10 Hz for 120 s; 1200 pulses)-induced calcitonin gene-related (CGRP) release from sensory fibres of the isolated rat tracheae. Each column represents the mean±s.e.m. CGRP concentration measured in the incubation medium of the prestimulated, stimulated and poststimulated 8-minute fractions of n = 5 experiments (5 x 2 tracheae/group). Analogs were added to the incubation medium (500 nM) at the beginning of the second and third fractions. **P*<0.05, ***P*<0.01, vs. respective fraction of the control experiment (Mann-Whitney U-test).

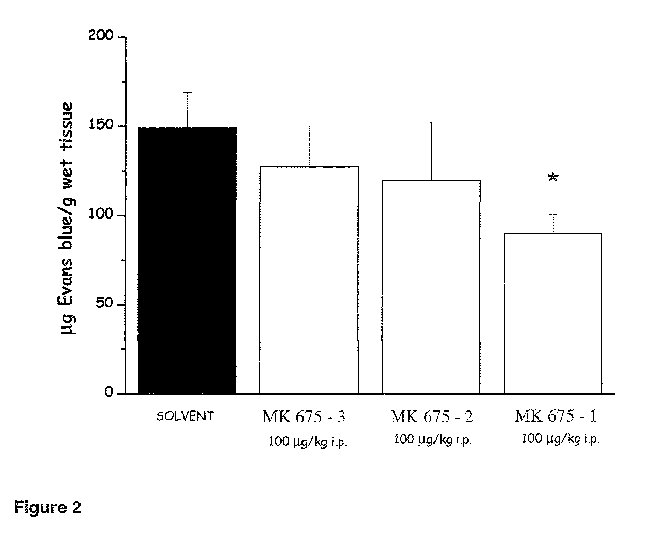

Figure 2 provides a graphical representation of the effect of small peptide somatostatin analogs on 1% mustard oil-induced acute neurogenic plasma protein extravasation in the dorsal skin of the rat paw. Each column represents the mean±s.e.m. Evans blue accumulation per g wet skin of *n* = 6-10 experiments. Analogs were injected i.p. (100 µg/kg) 10 min before the induction of the inflammation. **P*<0.05 vs. solvent-treated control experiment (Mann-Whitney U-test).

### 5. DETAILED DESCRIPTION OF THE INVENTION

### 5.1 Definitions

In the present application several abbreviated designations are used for the amino acid components, protecting groups, reagents and solvents. The meanings of such abbreviations are explained in Table 1.

**Table 1. Abbreviations and their meanings.**

| **Abbreviation** | **Amino acid** |
|---|---|
| Ala | L-alanine |
| Lys | L-lysine |
| Phe | L-phenylalanine |
| D-Trp | D-tryptophan |
| Tyr | L-tyrosine |
| Val | L-valine |

| **Abbreviation** | **Protecting group** |
|---|---|
| Boc | tert-butyloxycarbonyl |
| o-NBS | 2-nitrobenzenesulfonyl chloride |
| Fmoc | 9-Fluorenylmethoxycarbonyl |
| t-Bu | tertiary-butyl |

| **Abbreviation** | **Reagents** |
|---|---|
| DBU | 1,8-diazabicyclo[5,4,0]undec-7-ene |
| DIAD | diisopropylazodicarboxylate |
| DIEA | diisopropylethylamine |
| DPPA | diphenylphosphoric acid azide |
| HATU | *O*-(7-Azabenzotriazol-1-yl)-*N*,*N*,*N*',*N'*,-tetramethyluronium-hexafluorophosphate |
| HOAt | 1-hydroxy-7-azabenzotriazol |
| HOBt | 1-hydroxybenzotriazol |
| NaHCO₃ | sodium bicarbonate |
| Na₂SO₄ | sodium sulfate |
| *o*-NBS | *o*-nitrobenzenesulfonyl |
| PPh₃ | triphenylphosphine |
| TBTU | *O*-(1H-Benzotriazol-1-yl)-*N*,*N*,*N*',*N'*-tetramethyluronium-tetrafluoroborate |
| TES | triethylsilane |
| TFA | trifluoroacetetic acid |

| **Abbreviation** | **Solvent** |
|---|---|
| DCM | dichloromethane |
| DMF | *N*,*N*-dimethylformamide |
| EtOAc | ethyl acetate |
| MeOH | methanol |
| NMP | *N*-methylpyrrolidone |
| TFE | trifluoroethanol |
| THF | tetrahydrofuran |

| **Abbreviation** | **Miscellaneous** |
|---|---|
| ESI-MS | Electrospray ionization mass spectrometry |
| TCP | tritylchloridepolystyrene resin |
| SPPS | solid phase peptide synthesis |
| RP-HPLC | reversed phase-high performance liquid chromatography |

The expression "orthogonally protected" means in the context of the present invention the use of two or more protecting groups that are selected such that each of the protecting groups can be removed under conditions that do not affect the other protecting groups. Orthogonal protecting groups for peptide synthesis are described in T. W. Green, P. G. M. Wuts, Protective Groups in Organic Synthesis, Wiley-Interscience, New York, 1999.

### 5.2 Compounds of the present Invention

The compounds of the present invention have the structural formula (I): 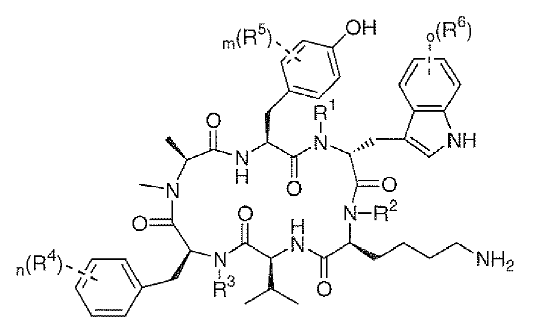
wherein R¹, R², R³ are each independently selected from hydrogen and C₁₋₆ alkyl groups, preferably hydrogen and C₁₋₃ alkyl groups, more preferably hydrogen and methyl groups, with the proviso that at least one of R¹, R², and R³ is not hydrogen; R⁴, R⁵ and R⁶ are substituents;
n denotes the number of substituents R⁴ present on the benzene ring and is selected from 0, 1, 2, 3, 4, and 5, preferably, 0, 1, 2, 3, and 4, more preferably 0, 1, 2, and 3, further preferably 0, 1, and 2, even more preferably 0 and 1, most preferably 0; if n is 2 or greater, the multiple substituents R⁴ may be the same or different;
m and o denote the respective numbers of substituents R⁵ and R⁶ and are independently selected from 0, 1, 2, 3, and 4, more preferably 0, 1, 2, and 3, further preferably 0, 1, and 2, even more preferably 0 and 1, most preferably 0; if m and/or o are 2 or greater, the multiple R⁵ and/or R⁶ may be the same or different,
and pharmaceutically acceptable salts, hydrates, solvates, polymorphs and pseudopolymorphs thereof.

In the above formula (I), it is prefered that m, n, and o are all independently selected from 0 and 1, and it is more preferred that m, n, and o are all 0 (i.e. no substituent R⁴, R⁵, R⁶ is present). Additionally, a compound of formula (I) wherein o and m are both 0 (R⁵ and R⁶ are not present) and wherein n is 1 is also preferred. In the latter cae, the one R⁴ is preferably halogen (preferably Cl or I), C₁₋₆ alkyl or C₁₋₆ alkoxy, preferably present in the 4-position of the ring.

The substituents represented by R⁴, R⁵ and R⁶, respectively, are not limited, and can be any group capable of substituting at the respective position.

Preferably, R⁴, R⁵ and R⁶ are independently selected from halogen (e.g. F, Cl, Br, I), alkyl groups (preferably having 1 - 6, more preferably 1 - 3 carbon atoms), alkoxy groups (preferably having 1 - 6, more preferably 1 - 3 carbon atoms), alkenyl groups (preferably having 2 - 6, more preferably 2 - 3 carbon atoms), alkinyl groups (preferably having 2 - 6, more preferably 2 - 3 carbon atoms), nitro, cyano, NR⁷R⁸ (wherein R⁷ and R⁸ are independently selected from hydrogen and C₁₋₆ alkyl), carboxyl, COR⁷, C(O)OR⁷, and OCOR⁷ (wherein R⁷ is in each case defined as above);
further preferably R⁴, R⁵ and R⁶ are independently selected from halogen (e.g. F, Cl, Br, I), alkyl groups (preferably having 1 - 6, more preferably 1 - 3 carbon atoms), alkoxy groups (preferably having 1 - 6, more preferably 1 - 3 carbon atoms), alkenyl groups (preferably having 2 - 6, more preferably 2 - 3 carbon atoms), NR⁷R⁸ (wherein R⁷ and R⁸ are independently selected from hydrogen and C₁₋₆ alkyl), carboxyl, COR⁷, C(O)OR⁷, and OCOR⁷ (wherein R⁷ is in each case defined as above);
more preferably R⁴, R⁵ and R⁶ are independently selected from halogen (e.g. F, Cl, Br, I), alkyl groups (preferably having 1 - 6, more preferably 1 - 3 carbon atoms), and alkoxy groups (preferably having 1 - 6, more preferably 1 - 3 carbon atoms);
and most preferalby R⁴, R⁵ and R⁶ are independently selected from halogen (e.g. F, Cl, Br, I, preferably Cl and I), and alkyl groups (preferably having 1 - 6, more preferably 1 - 3 carbon atoms).

If two or more R⁴, R⁵, and/or R⁶ are present (at least one of n, m, and o is 2 or greater), multiple neighboring R⁴, R⁵ and/or R⁶, respectively, may bind to each other to form a cyclic structure, preferably a saturated or unsaturated 5 - 7 (preferred 6) membered hydrocarbon ring, which may comprise one to two heteroatoms selected from O, S, and N.

The position of the substituents R⁴, R⁵, and R⁶ on the respective aromatic ring they bind to is not limited. However, R4 is preferably present in the 4-position (para to the bond to the remainder of the molecule).

Compounds wherein each of R¹, R², R³ independently represents hydrogen or CH₃ are particularly preferred, as these compounds are most suitable for the treatment of neurogenic inflammation (neuropathic pain). It is further preferred that in such a case n, m, and o are all 0, or that m and o are 0 and n is 1. In the latter case, it is preferable that the one R4 is halogen (in particular Cl or I), C₁₋₆ alkyl or C₁₋₆ alkoxy, further preferably halogen.

It is even more preferred that R¹ and/or R² represent a methyl group. It is even more preferred that each of R¹, R² and R³ represents a methyl group. It is further preferred that in such a case n, m, and o are all 0, or that m and o are 0 and n is 1. In the latter case, it is preferable that the one R4 is halogen (in particular Cl or I), C₁₋₆ alkyl or C₁₋₆ alkoxy, further preferably halogen.

The present application thus pertains inter alia to the following compounds:
i. cyclo(-MeAla-Tyr-D-MeTrp-MeLys-Val-MePhe-)
ii. cyclo(-MeAla-Tyr-D-MeTrp-MeLys-Val-Phe-)
iii. cyclo(-MeAla-Tyr-D-Trp-MeLys-Val-Phe-)
iv. cyclo(-MeAla-Tyr-D-MeTrp-Lys-Val-Phe-)
v. cyclo(-MeAla-Tyr-D-Trp-Lys-Val-MePhe-)
vi. cyclo(-MeAla-Tyr-D-Trp-MeLys-Val-MePhe-)
vii. cyclo(-MeAla-Tyr-D-MeTrp-Lys-Val-MePhe-)

Among these compounds, compound i. (cyclo(-MeAla-Tyr-D-MeTrp-MeLys-Val-MePhe-)) is particularly preferred.

The present invention also pertains to pharmaceutically acceptable salts of the cyclopeptides of formula (I). These salts can be acid addition salts derived from organic acids and/or inorganic acids. Typical salts include salts derived from acetic acid, tartaric acid, citric acid, trifluoro acetic acid, methanesulfonic acid, hydrochloric acid, sulfuric acid, nitric acid, phosphoric acid, and the like. The compounds of the present invention include in particular the pharmaceutically acceptable salts approved by FDA and/or EMEA. A review of pharmaceutically acceptable salts that can be used in accordance with the present invention has been published by Berge in J. Pharm. Sci., 66(1): 1-19 (Jan. 1977) "Pharmaceutical Salts".

The compounds of the present invention may be employed in liquid (dissolved, suspended) or solid form. If the compounds of the invention are in solid form, they may exist in different polymorphic or pseudopolymorphic states and/or in different hydrated or solvated forms. All of these polymorphic and pseudopolymorphic forms as well as all solvates and hydrates, and also the amorphous form are contemplated for use in accordance with the present invention.

### 5.3 Synthesis

The compounds of the invention can be synthesized by cyclising the corresponding linear peptides. The linear peptides are preferably synthesized on solid phase by the sequential synthesis technique (SPPS) [R.B. Merrifield (1963) J. Am. Chem. Soc. 85, 2149-2154]. Thus the synthesis of the cyclic peptides in the present investigation comprises preferably of
a) synthesis of the orthogonally protected linear peptides on solid support;
b) deprotection at the N-terminal to get the free amine;
c) cleavage of the linear peptide from the resin;
d) cyclization to get the linear peptides; and
e) removal of the orthogonal protecting groups.

In these peptides, owing to N-methylation L-Iysine is preferably taken as the C-terminal to couple to TCP resin [K. Barlos et al. (1991) Int. J. Pept. Protein Res. 37, 513-520]. This selection is advantageous to obtain efficient cyclization with maximum yield of the cyclized product.

N-methylation of the amino acids L-Ala and L-Phe may be carried out in solution by Freidinger's method [R.M. Freidinger et al. (1983) J. Org. Chem. 48, 77-81]. An advantageous variant of this process is described in the following: 5.0 g Fmoc-Phe-OH (12.9 mmol) are suspended in 250 mL toluene and 3.0 g paraformaldehyde (100 mmol) and 300 mg *p*-toluenesulfonic acid are added. The mixture is refluxed for 1 hour under azeotropic water removal. The solution is cooled and washed two times with 1 N aqueous NaHCO₃, dried and concentrated *in vacuo* to give the corresponding oxazolidinone in quantitative yields.

The oxazolidinone is dissolved in 100 mL DCM, and 100 mL TFA and 6.6 mL TES (3 eq.) are added and stirred at room temperature for 16 hours. After concentration in *vacuo,* the resultant oil is dissolved in ether and precipitated in hexane to give pure Fmoc-MePhe-OH in quantitative yields.

Similarly, 5.0 g of Fmoc-Ala-OH (16.0 mmol) are suspended in 300 mL toluene and 3.20 g paraformaldehyde (106.6 mmol) and 320 mg *p*-toluenesulfonic acid are added. The mixture is refluxed for 1 hour under azeotropic water removal. The solution is cooled and washed two times with 1 N aqueous NaHCO₃, dried and concentrated *in vacuo* to give the corresponding oxazolidinone in quantitative yields.

The oxazolidinone is dissolved in 120 mL DCM and 120 mL TFA to which 7 mL (3.18 eq) TES are added and stirred at room temperature for 16 hours. After concentration *in vacuo,* the resultant oil is dissolved in ether and precipitated in hexane to give pure Fmoc-MeAla-OH in quantitative yields.

To obtain N-methylated derivatives containing Me-Lys(Boc) and Me-Trp(Boc), an enhanced MITSUNOBU method may be used [E. Biron et al. (2005) J. Pept. Sci. 12, 213-9]. An advantageous variant of this process is described in the following: The peptide is synthesized up to the amino acid that is to be N-methylated and the Fmoc protection group is cleaved with 20% piperidine in NMP. The free N-terminal is protected using *o*-NBS group, which is achieved by treatment with 5 eq. 2-nitrobenzenesulfonyl chloride and 10 eq. Collidine in NMP for 15 min. N-Methylation is obtained by reaction with 5 eq. triphenylphosphine, 10 eq. dry MeOH and 5 eq. DIAD in dry THF for 10 minutes. Deprotection of the *o*-NBS group is achieved by treatment with 5 eq. DBU and 10 eq. mercaptoethanol in NMP twice for 5 minutes.

3 eq. of HATU, 3 eq. HOAt and 6 eq. DIEA are used for coupling of the following amino acid for 2 to 3 hours, which is monitored by using analytical RP-HPLC.

Peptide synthesis may be carried out using Tritylchloride polystyrene TCP-resin (0.94 mmol/g) following standard Fmoc-strategy using Fmoc-Lys(Boc)-OH, Fmoc-D-Trp(Boc)-OH, Fmoc-Tyr(t-Bu)-OH, Fmoc-MeAla-OH/ Fmoc-Ala-OH, Fmoc-MePhe-OH/ Fmoc-Phe-OH and Fmoc-Val-OH. Fmoc-Lys(Boc)-OH (1.2 eq.) may be attached to the TCP resin with DIEA (2.5 eq.) in anhydrous DCM (2 mL) at room temperature for 1.5 h. After filtration the remaining trityl chloride groups are capped by a solution of DCM, MeOH, DIEA (17:2:1; v:v:v) for 15 min. The resin is filtered and washed thoroughly with DCM (2x), NMP (3x) and MeOH (5x). The loading capacity is determined by weight after drying the resin under vacuum and ranges from 0.75-0.8 mmol/g. The Fmoc group of lysine is removed with 20% piperidine in NMP to give the free amino group, which is either N-methylated on solid support or is coupled to the next amino acid (Fmoc-D-Trp(Boc)-OH) using 2 eq. of the amino acid, 2 eq. of TBTU and HOBt and 6 eq. of DIEA for 1 hour.

Once the desired linear peptide is synthesized on solid support, it can be cleaved from the TCP resin by using a solution of acetic acid/2,2,2-trifluoroethane (TFE) mixture in dichloromethane (DCM) (3:1:6), which is repeated 3 times with an hour each.

The resultant solution with the peptide is co-evaporated with toluene to completely remove the acetic acid, which might cap the free N-terminal during cyclization resulting in lower yields. The sticky oil obtained is eventually dried overnight under high vacuum to get the crude linear peptide.

The cyclization is performed with HATU and HOBt with Collidine as a base [E. Biron et al. (2008) Angew. Chem. Int. Ed. 47, 2595-2599] under high dilution conditions to avoid any cyclodimerization and oligomerization. To a vigorously stirred solution of the linear peptide in DMF, a solution of HATU (1.5eq.), HOBt (1.5 eq.) and Collidine (1.5 eq.) in DMF is added dropwise making a final concentration of 5 mM. The solution is stirred overnight (14 hours) and then the DMF is evaporated to dryness. To the crude product is added a saturated solution of NaHCO₃ and extracted with EtOAc thrice. The organic layer is ished with brine and dried with Na₂SO₄. The EtOAc is evaporated finally to obtain the crude product free of cyclization reagents. The pure cyclized product is obtained finally by RP-HPLC.

The pure cyclized peptide is lyophilized to get the protected linear peptide in a powder form, which is dissolved in minimum volume of a 1:1 mixture of TFA:DCM (dry) and stirred for 1 hour to cleave the Boc and the t-Bu protecting groups, which is monitored by ESI-MS spectroscopy. The resultant solution is evaporated finally to 1 mL and is dropped into ether and centrifuged for 15 minutes at 4,000 rpm to give the pure deprotected peptide.

### 5.4 Formulation, Medical Uses and Administration

The compounds of the present invention can be used for the production of pharmaceutical compositions by bringing them into a suitable dosage form together with at least one pharmaceutically acceptable excipient or auxiliary and, if desired, together with one or more other active ingredients. Suitable excipients are organic or inorganic substances which are suitable for enteral (e.g. oral or rectal), parenteral (e.g. intravenous injection) or local (e.g. topical, dermal, ophthalmic or nasal) administration or for administration in the form of an inhalation spray, provided they do not react with the compounds with the present invention.

Typical examples of suitable excipients are water or aqueous isotonic saline solutions, lower alcohols (e.g. having 1 - 6 carbon atoms), vegetable oils, benzyl alcohols, polyethylene glycols, glycerol triacetate and other fatty acid glycerides, gelatine, soya lecithin, carbohydrates such as lactose or starch, magnesium stearate, talc, cellulose and petroleum jelly.

For oral application, plain tablets, coated tablets, capsules, syrups, juices or drops are particularly useful; coated tablets and capsules having enteric coating or capsule shells are especially of interest. Suppositories are used for rectal administration, solutions for parenteral administration, preferably oily or aqueous solutions and also suspensions, emulsions or implants. Examples of forms suitable for topical application are solutions, suspensions, emulsions, crèmes, ointments or compresses. For administration in the form of an inhalation spray it is possible to use sprays which contain the active ingredient either dissolved or suspended in propellant gas or propellant gas mixture (e.g. CO₂ or fluorochlorohydrocarbon substitutes). In this case, the active ingredient is expediently used in micronized form, with the presence of one or more additional physiologically compatible solvents such as ethanol being possible. Inhalation solutions can be administered with the aid of customary inhalers. The use of powder inhalers is also contemplated. For this purpose, the compounds of the invention are preferably employed in the form of solid particles having a suitable particle diameter of typically 1 to 5 µm. Such particles are preferably blended with inert carrier particles, typically lactose monohydrate.

The compounds of the invention can be administered in the form of injection preparations. Injections can be given as a bolus or in the form of a continuous infusion (for example intravenous, intramuscular, subcutaneous or intrathecal). Such preparations can be sterilized and/or can comprise auxiliaries such as preservatives, stabilizers and/or wetting agents, emulsifiers, salts for influencing osmotic pressure, buffer substances, colorants and/or flavorings.

If desired, the pharmaceutical compositions of the present invention can also contain one or more other active ingredients such as non-steroidal or steroidal anti-inflammatory drugs or analgesics.

Calcitonin gene-related peptide (CGRP) together with other pro-inflammatory neuropeptides released from sensory nerve terminals is responsible for the mediation neurogenic inflammation (vasodilatation and plasma protein extravasation) in different organs. Since the compounds of the present invention significantly inhibit CGRP release from isolated peripheral endings of primary afferents, they are likely to be active against neurogenic inflammatory processes, and they might also be appropriate in nociceptive conditions such as neuropathic pain. They are particularly suitable for the treatment of neurogenic inflammation and symptoms thereof including vasodilation and plasma protein extravasation (oedema formation). The neurogenic inflammatory component plays an important role in the pathological mechanisms of several diseases such as rheumatoid arthritis, allergic contact dermatitis, psoriasis, asthma and inflammatory bowel diseases, therefore, the compounds of the present invention can also be used to treat such diseases respectively patients suffering therefrom. This is particularly important, since the presently available classical non-steroidal anti-inflammatory/analgesic drugs are not able to diminish the neurogenic components of these inflammatory reactions and neuropathic pain.

Accordingly, the present invention also pertains to methods for the treatment of medical indications selected from neuropathic pain, neurogenic inflammation and symptoms thereof including vasodilation and plasma protein extravasation (oedema formation) as well as rheumatoid arthritis, allergic contact dermatitis, psoriasis, asthma and inflammatory bowel diseases in a subject in need thereof, comprising the administration of an effective amount of a compound of the present invention.

Preferred dosages of the compound of the present invention are between about 0.05 and 500 mg, more preferably between 0.5 and 100 mg, and most preferably between 1 and 50 mg, per dosage unit. The daily dose is preferably between about 0.01 and 2 mg/kg, more preferably between about 0.02 to 1 mg/kg of body weight. The appropriate dose for each individual patient can be determined by means of standard procedures, taking into account activity of the compound of the invention, age, body weight, general condition of health, gender of the patient and the like.

Formulation and administration of the compounds of the present invention can be effected also as described in US 4,472,305.

### 6. EXAMPLES

### 6.1 Preparation Examples

Three compounds of the invention (MK678_1 to MK678_3) were synthesized together with the above-mentioned compound of Veber *et al.* (MK678_0) as a comparative compound. The synthetic procedures described in Section 5.3 above were employed. Table 2 below summarizes the results of these experiments.

**Table 2. Synthesized peptides, their yields and characterization.**

| **Code** | **Compound** | **Cyclized yield** | **Mol Wt. (M+H)⁺** | **t*_{R}*(C18 column 10-100% ACN:H₂O₊ 0.1% TFA)** |
|---|---|---|---|---|
| MK678_3 (3) | c(-MeAYwMeKVF-) | 71% | 823.4 | 16.13 |
| MK678_2 (2) | c(-MeAYMewMeKVF-) | 68% | 837.4 | 16.87 |
| MK678_1 (1) | c(-MeAYMewMeKVMeF-) | 72% | 851.5 | 17.83 |
| MK678_0 (0) | *c(-MeAYwKVF-)* | 83% | 809.4 | 15.15 |

### 6.2 Activity Studies

***Ethics:** All experimental procedures were carried out according to the 1998*/*XXVIII Act of the Hungarian Parliament on Animal Protection and Consideration Decree of Scientific Procedures of Animal Experiments (243*/*1988) and complied with the recommendations of the Helsinki Declaration. The studies were approved by the Ethics Committee on Animal Research of Pécs University according to the Ethical Codex of Animal Experiments and license was given (license No.: BA 02*/*200-6-2001).*

### 6.2.1 Introduction in vitro Test

The activities of the above described synthetic somatostatin analogs were determined by means of an *in vitro* pharmacological assay on the release of the sensory neuropeptide CGRP from the peripheral terminals of sensory nerves of the isolated rat trachea. This experimental model was established more than 10 years ago (Helyes et al. (1997) Br. J. Pharmacol. 121, 613-615; Szolcsányi et al. (1998) Br. J. Pharmacol. 123, 936-942; Szolcsányi et al. (1998) Br. J. Pharmacol. 125, 916-922; Helyes et al. (2004) Arthr. Rheum., 50, 1677-1685; Bölcskei et al. (2005) Pain 117, 368-376; Helyes et al. (2007) Am. J. Physiol. Lung Cell. Mol. Physiol. 292, L1173-81; Nemeth et al. (1998) Eur. J. Pharmacol, 347, 101-104.) and it represents a reliable and sensitive technique for the investigation of drug effects at the level of peripheral nerve endings. Electrical field stimulation parameters are used to induce CGRP release that is selective for capsaicin-sensitive afferents. Since CGRP is an important mediator of neurogenic inflammation, the effectiveness of compounds in decreasing its electrically-evoked release *in vitro* may be seen as an indicator for effectiveness to inhibit neurogenic inflammatory reactions *in vivo.*

### 6.2.2 Methods in vitro Test

Experiments were performed on male Wistar rats bred and kept in the Laboratory Animal Centre of the University of Pécs under standard pathogen free conditions at 24-25 °C and provided with standard rat chow and water *ad libitum.* The method has been described in detail elsewhere (Helyes et al. (1997) Br. J. Pharmacol. 121, 613-615; Nemeth et al. (1998) Eur. J. Pharmacol. 347, 101-104.). In brief, rats were exsanguinated in deep anesthesia (sodium thiopental, 50 mg/kg i.p.), then the whole trachea was removed and cleaned of fat and adhering connective tissues. Tracheae from two rats were placed into the same organ bath (1.8 ml) to achieve sufficient amount of peptide release and perfused (1 ml/min) with pH- (7.2) controlled oxygenized Krebs solution for 60 min (equilibration period) at 37 °C temperature. After discontinuation of the flow, the solution was changed three times for eight minutes to produce prestimulated, stimulated, poststimulated fractions. In the second 8-min period electrical field stimulation (EFS; 40 V, 0.1 ms, 10 Hz for 120 s; 1200 pulses) was performed to elicit neurotransmitter release. Concentration of CGRP was determined from 200 µL samples of the incubation medium by a selective and specific radioimmunoassay (RIA) technique as described previously in detail (Nemeth *et al.,* 1996; Helyes *et al.,* 1997; Nemeth *et al.,* 1998; Nemeth *et al.,* 1999). Peptide outflow was expressed as the released amount per wet tissue weight. Detection limit of the assay was 0.2 fmol/tube. For the specific RIA assays the antiserum ("C1012") was raised in rabbits with synthetic peptides conjugated to thyroglobulin by carbodiimide. The tracers were mono-¹²⁵I-labelled peptides prepared in our laboratory. Synthetic peptides were used as RIA standards ranging from 0 to 1000 fmol/ml. The assay was prepared in 1 mL 0.05 mol/l (pH 7.4) phosphate buffer containing 0.1 mol/L sodium chloride, 0.25 % (w/v) BSA and 0.05 % (w/v) sodium azide. The antiserum (100 µL, 1:10000 dilution), the RIA tracer (100 µL 5000 cpm/tube) and the standard or unknown samples (100 µL) were measured into polypropylene tubes with the assay buffer. After 48-72 h incubation at 4°C, the antibody-bound peptide was separated from the free one by addition of 100 µL separating solution (10 g charcoal, 1 g dextran and 0.5 g commercial fat-free milk powder in 100 mL distilled water). Following centrifugation (3000 rpm, 4 °C, 15 min) the tubes were gently decanted and the radioactivity of the precipitates was measured in a gamma counter (Gamma, type: NZ310). Peptide concentrations of the unknown samples were read from a calibration curve.

The examined peptides were added into the incubation medium at the beginning of the second and third 8-min fraction in 500 nM concentration. Each peptide was tested in separate experiments, only one peptide was applied to the same tracheae. In each group 5 experiments were performed to provide n=5 data points per group (10 tracheae per group). Results were evaluated by the non-parametric Mann Whitney U-test (unpaired comparisons), p<0.05 was considered significant.

### 6.2.3 Results in vitro Test

In control experiments EFS (1200 pulses) evoked an approximately 5-fold and 3.5-fold increase in the outflow of CGRP in the stimulated and post-stimulated fractions, respectively, compared to the basal release. This was significantly inhibited in the presence of 500 nM MK-675-3, MK-675-2 and MK-675-1. The latter compound induced the greatest, about 70% inhibitory action in the stimulated period and completely abolished CGRP outflow in the poststimulated fraction (Fig. 1). The same inhibitory actions are seen for these three analogs if the total CGRP release in the stimulated and poststimulated fractions together are calculated as percentage values compared to the respective basal outflow (Table 3).

**Table 3.**

| **Treatment (500 nM)** | **CGRP release in % compared to the prestimulated fraction** |
|---|---|
| Control | 405,02 ± 57,46 |
| MK-678-1 | 99,58 ± 39,89** |
| MK-678-2 | 153,12 ± 29,72* |
| MK-678-3 | 181,57 ± 54,17* |
| MK-678-0 | 293,96 ± 33,12 |

### 6.2.4 Introduction in vivo Test

To confirm the findings of this in vitro study, a further *in vivo* activity test was performed: Compounds of the invention were examined on 1% mustard oil-induced neurogenic plasma protein extravasation in the rat paw skin. This concentration of mustard oil has been shown to selectively stimulate capsaicin-sensitive nerves and induce a purely neurogenic acute inflammatory reaction.

### 6.2.5 Methods in vivo Test

Both hindlegs of the rats were acutely denervated (the sciatic and the saphenous nerves were cut 30 min before the induction of inflammation) under sodium pentobarbitone (Nembutal, 40 mg/kg i.p.) anaesthesia to avoid central reflexes. Acute neurogenic inflammation in the dorsal skin of the hindpaws was evoked by topical application of 1 % mustard oil dissolved in paraffin oil. Extravasation of plasma albumin was measured by the Evans blue leakage method. Evans blue (50 mg/kg) was injected i.v. and neurogenic inflammation was induced 10 min later. Rats were killed by exsanguination 20 min after mustard oil application. The skin of the hindpaws was removed and the extravasated dye was extracted with formamide for 72 h at room temperature for photometric determination at 620 nm. The amount of the accumulated Evans blue, which quantitatively correlates with the intensity of plasma extravasation, was expressed as µg dye/g wet tissue (Helyes *et al.,* 1997; Helyes *et al.,* 2001). Each compound (100 µg/kg) was administered i.p. 10 min before the induction of the inflammation. For comparison, the solvent was administered instead of the analogs. This study was undertaken in blocks with 14-15 rats per occasion. The whole set of data was obtained during 3 days. There were 2 or 3 solvent-treated rats every day and the remaining 12 animals were randomised to receive each treatment.

### 6.2.6 Results in vivo Test

Intraperitoneal injection of 100 µg/kg MK 675-1 induced a significant, about 40% inhibitory action on 1% mustard oil-evoked acute neurogenic plasma protein extravasation in the rat paw skin. The other two tested compounds of the invention gave rise to smaller inhibitory effects.

| | **µg Evans blue/g wet tissue** **(mean±s.e.m.)** | **% inhibition compared to the control** |
|---|---|---|
| SOLVENT | **148.95±20.1** | |
| MK 675-3 100 mg/kg i.p. | **127.13±23.0** | 14.16 |
| MK 675-2 100 mg/kg i.p. | **119.92±32.7** | 19.57 |
| MK 675-1 100 mg/kg i.p. | **90.06±10.4*** | 39.54 |

| | | |
|---|---|---|
| *p<0.05 Mann-Whitney U test | | |

## Claims

1. Cyclopeptide having the following formula (I): 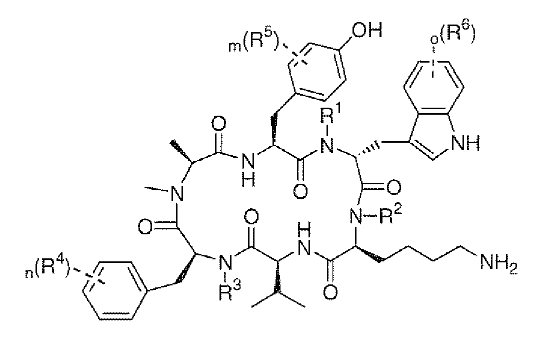
wherein R¹, R², R³ are each independently selected from hydrogen and C₁₋₆ alkyl groups, preferably hydrogen and C1-3 alkyl groups, more preferably hydrogen and methyl groups, with the proviso that at least one of R¹, R², and R³ is not hydrogen; R⁴, R⁵ and R⁶ are substituents;
n denotes the number of substituents R⁴ present on the benzene ring and is selected from 0, 1, 2, 3, 4, and 5, preferably, 0, 1, 2, 3, and 4, more preferably 0, 1, 2, and 3, further preferably 0, 1, and 2, even more preferably 0 and 1, most preferably 0; if n is 2 or greater, the multiple substituents R⁴ may be the same or different;
m and o denote the respective numbers of substituents R⁵ and R⁶ and are independently selected from 0, 1, 2, 3, and 4, more preferably 0, 1, 2, and 3, further preferably 0, 1, and 2, even more preferably 0 and 1, most preferably 0; if m and/or o are 2 or greater, the multiple R⁵ and/or R⁶ may be the same or different;
and pharmaceutically acceptable salts, hydrates, solvates, polymorphs and pseudopolymorphs thereof.

2. Cyclopeptide, and pharmaceutically acceptable salts, hydrates, solvates, polymorphs and pseudopolymorphs thereof according to Claim 1, wherein R¹, R², R³ are each independently selected from hydrogen and a methyl group.

3. Cyclopeptide and pharmaceutically acceptable salts, hydrates, solvates, polymorphs and pseudopolymorphs thereof according to Claim 2, wherein R¹ and/or R² represents a methyl group.

4. Cyclopeptide and pharmaceutically acceptable salts, hydrates, solvates, polymorphs and pseudopolymorphs thereof according to anyone of the preceding claims, wherein each of R¹, R², and R³ represents a methyl group.

5. Pharmaceutical composition comprising the cyclopeptide, pharmaceutically acceptable salts, hydrates, solvates, polymorphs and pseudopolymorphs thereof, according to anyone of Claims 1 to 5, and a pharmaceutically acceptable excipient.

6. Cyclopeptide and pharmaceutically acceptable salts, hydrates, solvates, polymorphs and pseudopolymorphs thereof according to anyone of Claims 1 to 5, for use in medicine.

7. Cyclopeptide and pharmaceutically acceptable salts, hydrates, solvates, polymorphs and pseudopolymorphs thereof according to anyone of Claims 1 to 5, for use in the treatment of pain.

8. Cyclopeptide and pharmaceutically acceptable salts, hydrates, solvates, polymorphs and pseudopolymorphs thereof according to anyone of Claims 1 to 5, for use in the treatment of neurogenic inflammation and/or neuropathic pain.

9. Cyclopeptide and pharmaceutically acceptable salts, hydrates, solvates, polymorphs and pseudopolymorphs thereof according to anyone of Claims 1 to 5, for use in the treatment of a medical indication selected from rheumatoid arthritis, allergic contact dermatitis, psoriasis, asthma and inflammatory bowel diseases.

10. Use of the cyclopeptide and pharmaceutically acceptable salts, hydrates, solvates, polymorphs and pseudopolymorphs thereof according to anyone of Claims 1 to 5, for the manufacture of a medicament for the treatment of pain and preferably neuropathic pain.

11. Process for the manufacturing of the cyclopeptide and pharmaceutically acceptable salts, hydrates, solvates, polymorphs and pseudopolymorphs thereof according to anyone of Claims 1 to 5, comprising the steps of
(a) synthesis of a linear peptide having the same amino acid sequence and N-alkylation pattern as the target compound on a solid support;
(b) deprotection of the linear peptide of step (a) at the N-terminal to obtain the linear peptide of the same amino acid sequence and N-alkylation pattern, but having a free terminal amino group;
(c) cleavage of the linear peptide obtained in step (b) from the resin;
(d) cyclisation of the cleaved linear peptide obtained in step (c) to get the cyclic peptide; and
(e) removal of the orthogonal protecting groups from the cyclic peptide of step (d) to obtain the cyclic peptide of formula (I).

12. Process according to Claim 11, wherein the synthesis of the linear peptide in step (a) starts with the coupling of L-lysine to the solid support.
